## Europäisches Patentamt
## European Patent Office
## Office européen des brevets

(11) Veröffentlichungsnummer: **0 000 133 B1**

(12) **EUROPÄISCHE PATENTSCHRIFT**

(45) Veröffentlichungstag der Patentschrift: **16.09.81**

(21) Anmeldenummer: **78100133.4**

(22) Anmeldetag: **09.06.78**

(51) Int. Cl.³: **A 61 K 33/30** //(A61K33/30, 31/25, 31/725)

(54) **Pharmazeutische Präparate mit einem Gehalt an sulfatierten Polysacchariden oder Polymeren und Zinkionen zur topischen Behandlung von Virusinfektionen.**

(30) Priorität: **17.06.77 LU 77562**

(43) Veröffentlichungstag der Anmeldung:
**10.01.79 Patentblatt 79/1**

(45) Bekanntmachung des Hinweises auf die Patenterteilung:
**16.09.81 Patentblatt 81/37**

(84) Benannte Vertragsstaaten:
**BE CH DE FR GB NL**

(56) Entgegenhaltungen:
**CHEMICAL ABSTRACTS, 66, 45191s (1967) zitiert "Acta Microbiol. Acad. Sci. Hung." 13 (3), 197—203 (1966)**

(73) Patentinhaber: **CIBA-GEIGY AG**
**Patentabteilung Postfach**
**CH-4002 Basel (CH)**

(72) Erfinder: **Lukas, Bohumir, Dr.**
**Laufenburgerstrasse 10/3**
**CH-4058 Basel (CH)**
Erfinder: **Wiesendanger, Walter**
**Steingrubenweg 12**
**CH-4142 Münchenstein (CH)**
Erfinder: **Schmidt-Ruppin, Karl Heinz, Dr.**
**Im Baumgarten 5**
**CH-4144 Arlesheim (CH)**

Courier Press, Leamington Spa, England.

**0 000 133**

Pharmazeutische Präparate mit einem Gehalt an sulfatierten Polysacchariden oder Polymeren und Zinkionen zur topischen Behandlung von Virusinfektionen

Die Erfindung betrifft neue pharmazeutische Präparate zur topischen Behandlung von Virusinfektionen, die eine antiviral wirksame Kombination eines sulfatierten Polysaccharids oder Polymeren, insbesondere Heparin, und Zinkionen in Form von dissoziierbaren Zinkverbindungen enthalten.

F. Lebel und Gy. Hadhazy berichteten in Acta Microbiol. Acad. Sci. Hung. 13(3) 197—203 (1966) [Chem. Abstr. 66, 45191s (1987)] über Versuche betreffend die hemmende Wirkung von exogenem Heparin auf das Wachstum von verschiedenen Stämmen von Herpes simplex-Virus an Kaninchen. Das Wachstum von 5 von 6 geprüften Stämmen und die Entwicklung von Hautläsionen wurde durch kutane Injektion von 1,2 mg Heparin 1 bis 18 Stunden vor der Infektion durch die zu prüfenden Stämme gehemmt.

Es wurde nun überraschenderweise gefunden, dass sulfatierte Polysaccharide oder Polymere, insbesondere Heparin, und Zinkionen in Form von dissoziierbaren Zinkverbindungen zusammen eine starke antivirale Wirkung ausüben, welche die Summe der Wirkungen der Einzelkomponenten weit übertrifft. Die synergistische Wirkung der beiden Komponenten lässt sich in vitro, d.h. in Zellkulturen, z.B. auf Grund der Virus-Inaktivierung, der Hemmung der Plaquebildung und der Virus-Replikation nachweisen:

1. *Inaktivierung von Herpes-Virus hominis*

Die Präparate wurden in bidestilliertem Wasser in abgestuften Konzentrationen gemischt, mit HVH2/Ang. beimpft und während 1 Stunde bei 35°C inkubiert. Die Bestimmung des Virusgehaltes in den Kontaktgemischen erfolgte auf Hühnerembryofibroblasten im Plaquetest. Die Proben wurden in Hanks'-Lösung+0,05% Albumin verdünnt auf Zellmonolayer von Hühnerembryofibroblasten gegeben. Nach 90 Minuten Virusabsorption wurden die Kulturen 2-mal gewaschen, mit 4 m LY-Agaroverlay [HanksLösung mit 0,5% Lactalbuminhydrolysat und 0,01% Yeastolate (Hefeextrakt)] versetzt und dann bis zur Zählung der Plaques (96 Stunden) bei 35°C inkubiert.

TABELLE I

| Präparate, Konzentrationen in g.$10^{-6}$/ml | | | |
|---|---|---|---|
| | Heparin *) | | |
| ZnSO$_4$ .7 H$_2$O | 0 | 33 | 100 |
| 0 | 5,10 [1] | 5,04 | 5,00 |
| 33 | 4,89 | 4,76 | 4,95 |
| 100 | 4,76 | 2,60 | 2,62 |
| 300 | 4,68 | <1,00 | <1,00 |

1) Virusgehalt: Log 10 PFU /ml (Plaque Forming Units /ml)

*) 160 IE /mg (von Biofac AS, Kopenhagen, Dänemark)

2. *Hemmung der Plaquebildung von HVH2/Ang. in Hühnerembryofibroblasten*

Die Zellmonolayer wurden mit 100 PFU HVH2/Ang. während 90 Minuten infiziert. Je 4 ml der in LY-Agaroverlay (mit 5% Schafserum und 0,5% OXO-L-28-Agar, einem gereinigten Agar der Oxoid Ltd., Wade Road, Basingstoke, England, ohne Diäthylaminoäthyldextran) inkorporierten Präparate wurden auf die infizierten Zellkulturen gegeben und diese bis zur Zählung der Plaques (96 Stunden) bei 35°C inkubiert.

## 0 000 133

TABELLE II

| Präparate, Konzentrationen in $g.10^{-6}$/ml | | | | |
|---|---|---|---|---|
| | Heparin *) | | | |
| $ZnSO_4.7H_2O$ | 0 | 1,5 | 3 | 6 |
| 0 | 100 [1]<br>2–3 [2] | 82<br>2–3 | 87<br>2–3 | 76<br>1–2 |
| 3 | 104<br>1–3 | 65<br>1–2 | 57<br>0,5–1 | 42<br>0,5 |
| 6 | 64<br>1–2 | 31<br>0,5–1 | 11<br>0,5 | 2<br>0,5 |
| 12 | 43<br>0,5–1 | 8<br>0,5 | 2<br>0,5 | 2<br>0,5 |
| 25 | 15<br>0,5 | 5<br>0,5 | 2<br>0,5 | 1<br>0,5 |

[1] Plaquezahl von 3 Schalen in %

[2] Plaquegrösse $\phi$ in mm (ca.)

*) 160 IE/mg (Biofac)

3. *Hemmung der Replikation von HVH2/Ang. in VERO-Zellen bei präinfektiösem Behandlungsbeginn*
Konfluente Monolayer von VERO-Zellen (Affennierenzellen, Cell Repository No. CGL 81 der American Type Culture Collection) wurde mit F15-Medium (Minimum Essential Medium nach Eagle von der Gibco Biocult Ltd., Paisley, Schottland) gewaschen und dann mit je 4 ml der verschiedenen Konzentrationen der Präparate in F15 Medium belegt. Die Infektion der Kulturen erfolgte nach 60 Minuten mit 1000 PFU HVH2/Ang. in 0,1 ml Medium. Nach 20 Stunden Inkubation bei 35°C wurde der Zustand der Zelle mikroskopisch beurteilt und der Virusgehalt der Zell-Lysate (Zellen mit 1 ml bindestilliertem Wasser 2-mal tiefgefroren und getaut) durch Titration auf Hühnerembryofibroblasten (Plaquebildung) bestimmt.

TABELLE III

| Präparate, Konzentrationen in g.$10^{-6}$/ml | | |
|---|---|---|
| | Heparin *) | |
| ZnSO$_4$.7H$_2$O | 0 | |
| 0 | 4,58 [1) | 1,84 |
| 6 | 3,59 | <1,00 |
| 12 | 3,32 | <1,00 |
| 25 | 2,82 | <1,00 |

1) Log 10 PFU /ml Zell-Lysat

*) 160 IE /mg (Biofac)

*Schlussfolgerungen aus den Resultaten der drei Versuchsreihen*

In den obigen Versuchsanordnungen ist die synergistische Wirkung der erfindungsgemässen Kombination von Heparin und Zinksulfat deutlich erkennbar. Besonders bemerkenswert ist bei der Virus-Inaktivierung gemäss Tabelle I, dass jede Komponente allein in allen geprüften Konzentrationen praktisch unwirksam war, während die Kombination von je einem Drittel der Maximalkonzentration der einzelnen Konponenten den Virusgehalt der Hühnerembryrofibroplasten bereits um $10^2$ verminderte. Aus Tabelle II ergibt sich u.a., dass Heparin allein nur schwach wirksam ist, jedoch sowohl Heparin als auch Zinksulfat-heptahydrat in der niedrigsten Konzentration zusammen mit jeder Konzentration der zweiten Komponente eine höhere Wirkung ergeben als die nächsthöhere Konzentration der zweiten Komponente allein. Mit der Kombination von 6.$10^{-6}$ g/ml Zinksulfat-heptahydrat und 3.$10^{-6}$ g/ml Heparin wird bereits die Wirkung von 25.$10^{-6}$ ml Zinksulfat-heptahydrat allein, und mit je 6.$10^{-6}$ g/ml Zinksulfat-heptahydrat und Heparin wie auch mit 12.$10^{-6}$ g/ml Zinksulfatheptahydrat und 3.$10^{-6}$ g/ml Heparin nahezu die Wirkung der Kombination von 25.$10^{-6}$ g/ml Zinksulfat-heptahydrat und 6.$10^{-6}$ g/ml Heparin erreicht. Die Virus-Replikation wird gemäss Tabelle III von Zinksulfat in der höchsten Konzentration von 25.$10^{-6}$ g/ml nur mässig, dagegen von Heparin in der einzigen geprüften Konzentration von 6.$10^{-6}$ g/ml, entsprechend der höchsten in den andern Versuchsreihen, infolge der starken Absorptionshemmwirkung des Heparin deutlich gehemmt; alle geprüften Kombinationen zeigen indessen eine noch mindestens 10-mal stärkere Wirkung als die Einzelkomponenten.

Ebenfalls festgestellt werden kann der synergistische Effekt der erfindungsgemäss kombinierten Wirkstoffe und zugleich die überlegene therapeutische Wirkung der Kombination im Vergleich zu andern, topisch anwendbaren antiviralen Präparaten in vivo bei der durch Infektion mit HVH2/Ang. verursachten Herpes genitalis des Meerschweinchens bei 72 Stunden nach Infektion (Stadium deutlicher Symptome) beginnender Behandlung, wobei auf die von B. Lukas et al., Arch. Ges. Virusforsch. *44*, 153—155 (1974) und *49*, 1—11 (1975) beschriebene Methodik hingewiesen wird. Die therapeutische Wirkung der Kombination ist nicht nur stärker, sondern tritt auch früher ein als die Wirkung der einzelnen Komponenten, überdies ist die Zahl der Rezidive stark vermindert.

Die synergistische Wirkung der obengenannten Wirkstoffe wird noch verstärkt, wenn eine Präparate-grundlage, insbesondere Gelgrundlage, verwendet wird, die einen oder mehrere Polyoxyäthlensorbitanfettsäureester, wie Polyoxyäthylensorbitan-monostearat und/oder vor allem Polyoxyäthylensorbitan-monolaurat oder in erster Linie Poloyoxyäthylensorbitan-monooleat, enthält.

Die erfindungsgemässen pharmazeutischen Präparate enthalten die oben definierten Wirkstoffe vorzugsweise in Kombination mit für die topische Applikation geeigneten pharmazeutischen Trägerstoffen. Als Formen von erfindungsgemässen Präparaten kommen insbesondere Tinkturen, Lösungen, Cremes, Salben und vor allem Gele in Betracht.

Tinkturen und Lösungen weisen meistens eine wässerig-äthanolische bzw. wässerige Grundlage auf, der u.a. Polyalkohole, wie z.B. Propylenglykol oder Glycerin und/oder niedrige Polyäthylenglykole, als Feuchthaltemittel zur Herabsetzung der Verdunstung, und gegebenenfalls rückfettende Substanzen, wie fettsäureester von niedrigen Polyäthylenglykolen, d.h. im wässerig-äthanolischen Gemisch lösliche, lipophile Substanzen, als Ersatz für die der Haut mit dem Aethanol entzogenen Fettsubstanzen, und gegebenenfalls weitere Hilfs- und Zusatzstoffe, neben üblichen, wie den untengenannten, Konservierungsmitteln z.B. auch die bereits erwähnten Polyoxyäthylensorbitan-fettsäureester, wie Polyoxyäthylensorbitan-monolaurat oder Polyoxyäthylensorbitanmonooleat, zugegeben sind.

Cremes sind Oel-in-Wasser-Emulsionen, die mehr als 50% Wasser aufweisen. Als ölige Grundlage verwendet man in erster Linie Fettalkohole, z.B. Lauryl-, Cetyl- oder Stearylalkohol, Fettsäuren, z.B. Palmitin- oder Stearinsäure, flüssige bis feste Wachse, z.B. Isopropylmyristinat, Wollwachs oder Bienenwachs, und/oder Kohlenwasserstoffe, z.B. Vaseline (Petrolatum) oder Paraffinöl. Als Emulgatoren kommen oberflächenaktive Substanzen mit vorwiegend hydrophilen Eigenschaften in Frage, wie entsprechende nichtionische Emulgatoren, z.B. Fettsäureester von Polyalkoholen oder Aethylenoxidaddukte davon, wie Polyglycerinfettsäureester oder Polyoxyathylen-sorbitan-fettsäureester (Tweens), ferner Polyoxyäthylenfettalkoholäther oder -fettsäureester, oder entsprechende ionische Emulgatoren, wie Alkalimetallsalze von Fettalkoholsulfaten, z.B. Natriumlaurylsulfat, Natriumcetylsulfat oder Natriumstearylsulfat, die man üblicherweise in Gegenwart von Fettalkoholen, z.B. Cetylakohol oder Stearylalkohol, verwendet. Zusätze zur Wasserphase sind u.a. Mittel, welche die Austrocknung der Creme vermindern, z.B. Polyalkohole, wie Glycerin, Sorbit, Propylenglykol und/oder Polyäthylenglykole, ferner Konservierungsmittel und Riechstoffe.

Salben sind Wasser-in-Oel-Emulsionen, die bis zu 70%, vorzugsweise jedoch von etwa 20% bis etwa 50% Wasser oder wässrige Phasen enthalten. Als Fettphase kommen in erster Linie Kohlenwasserstoffe, z.B. Vaseline, Paraffinöl und/oder Hartparaffine in Frage, die zue Verbesserung des Wasserbindungsvermögens vorzugsweise geeignete Hydroxyverbindungen, wie Fettalkohole, oder Ester davon, z.B. Cetylalkohol oder Wollwachsalkohole, bzw. Wollwachs enthalten. Emulgatoren sind entsprechende lipophile Substanzen, wie Sorbitan-fettsäureester (Spans), z.B. Sorbitanoleat und/oder Sorbitaniosostearat. Zusätze zur Wasserphase sind u.a. Feuchthaltungsmittel, wie Polyalkohole, z.B. Glycerin, Propylenglykol, Sorbit und/oder Polyäthylenglykol, sowie Konservierungsmittel und Riechstoffe.

Gele sind insbesondere wässrige Lösungen der Wirkstoffe, in denen Gelbildner, vorzugsweise solche aus der Gruppe der Celluloseäther, wie z.B. Methylcellulose, Hydroxyäthylcellulose oder Carboxymethylcellulose, oder der pflanzlichen Hydrokolloide, wie Natrium-alginat, Traganth oder Gummi arabicum, dispergiert und ausgequollen sind. Weiter enthalten die Gele vorzugsweise ebenfalls Feuchthaltemittel aus der Gruppe der Polyalkohole, wie Propylenglykol, Glycerin und/oder niedrige Polyäthylenglykole, sowie Netzmittel, z.B. Polyoxyäthylensorbitan-fettsäureester, wie Polyoxyäthylensorbitanmonostearat, -monolaurat oder -monooleat in Konzentrationen von ca. 0,02 bis 5%. Als weitere Zusatzstoffe enthalten die Gele übliche Konservierungsmittel, z.B. Benzylakohol, Phenäthylalkohol, Phenoxyäthanol, p-Hydroxybenzoesäureniederalkylester wie der Methyl- und/oder Propylester, Sorbinsäure oder organische Quecksilberverbindungen, wie Merthiolat.

Die erfindungsgemässen Präparate können — neben den üblichen Konservierungsmitteln — auch weitere biologisch, z.B. antiphlogistisch oder antimikrobiell, wie antibakteriell, antifungal oder ebenfalls antiviral wirksame Stoffe, wie z.B. Flumethason, Neomycin, Gentamycin, Milchsäure oder Mikonazol enthalten. Die Herstellung der erfindungsgemässen Präparate erfolgt in an sich bekannter Weise.

Die vorliegende Erfindung betrifft topisch anwendbare antivirale Präparate, die sulfatierte Polysaccharide oder Polymere, wie Heparin, und Zinkionen in einem Verhältnis von 1 mg zu 0,18 bis 18 mg, insbesondere von 1 mg zu 0,18 bis 4,5 mg, und gegebenenfalls Polyoxyäthylensorbitanmonolaurat und oder -monooleat enthalten. Für Heparin beziehen sich obige Mengenangaben auf solches mit 160 IE/mg, von anderem Heparin sind gleiche IE-Mengen einzusetzen. Die Zinkionen werden in Form der entsprechenden Mengen einer dissozierbaren Zinkverbindung, z.B. von 0,8—80 mg bzw. 0,8—20 mg $ZnSO_4.7H_2O$, zugefügt. Entsprechende topisch anwendbare Präparate, insbesondere Gele, ferner auch Tinkturen, wässrige Lösungen, Cremen oder Salben, enthalten beispielsweise pro g oder ml 0,1 bis 5 mg eines sulfatierten Polysacchariads oder Polymeren, insbesondere 16 bis 800 IE Heparin, und 0,18 bis 18 mg Zinkionen, entsprechend z.B. ca. 0,8 bis 80 mg $ZnSO_4.7H_2O$, und gegebenenfalls zusätzlich 0,2 bis 50 mg Polyoxyäthylensorbitan-monolaurat und/oder -monooleat. Besonders bevorzugt ist ein Gehalt von 80—320 IE Heparin, 0,45 bis 4,5 mg Zinkionen und gegebenenfalls zusätzlich 0,5 bis 10 mg Polyoxyäthylensorbitan-monolaurat und/oder -monooleat pro g oder ml.

Anstelle des Heparins kann auch eine antiviral wirkungsgleiche Menge eines andern sulfatierten Polysacchariads oder Polymeren, wie von natürlichen oder partiell abgebauten, sulfatierten Polysacchariden, wie sulfatierten Amylopectinen, sulfatierten Dextranen, sulfatierten Polyglucosen oder sulfatierten Polypentosen, bzw. von Polyvinylsulfaten, wie beispielsweise das Natriumsalz des Calciumkomplexes der Sulfatationsprodukte von oxidativ abgebauten Polygalakturonsäure-methylestern [Wirkstoff von HEMERAN (Geigy)] verwendet werden. Die Zinkionen können, statt in Form von Zinksulfat, auch in Form einer andern dissozierbaren Zinkverbindung, wie z.B. Zinkchlorid, oder des Zinksalzes einer Säure oder eines andern Stoffes von saurem Charakter und eigenen biologischen, z.B. antibakteriellen oder antiphlogistischen, Eigenschaften, wie z.B. Zink-sudoxicam (Zinksalz des 4 - Hydroxy - 2 - methyl - N - (2 - thiazolyl) - 1,2 - benzothiazin - 3 - carboxamid - 1,1 - dioxid) zugefügt werden.

Die erfindungsgemässen Präparate eignen sich insbesondere zur Behandlung von Herpes genitalis, Herpes dermatitis und Herpes labialis.

Das nachfolgende Beispiel beschreibt die Herstellung einer typischen Applikationsform.

Beispiel

Zur Herstellung von 10,0 Liter Gel vermischt man 200,0 g hochvisköse Natrium-carboxymethylcellulose und 50,0 g Polyoxyäthylensorbitan-monostearat (TWEEN 60) mit 1000 g Glycerin und 6,5 Liter Aqua conservans und lässt das Gemisch zu einem homogenen Schleim ausquellen. Dann wird eine Lösung von $1,6.10^6$ internationalen Einheiten Heparin (z.B. 10,0 g Heparin Biofac), 100,0 g Zinksulfatheptahydrat und 10,0 g Polyoxyäthylensorbitan-monooleat (TWEEN 80) in 2.0 Liter Aqua conservans zugemischt. Schliesslich wird mit Aqua conservans auf 10,0 Liter ergänzt, sorgfältig gemischt und das erhaltene Gel in Tuben abgefüllt.

Unter Aqua conservans wird eine wässrige Lösung von 0,07% p-Hydroxy-benzoesäure-methylester (Methylparaben) und 0,03% p-Hydroxybenzoesäure-propylester (Propylparaben) verstanden. TWEEN 60 und TWEEN 80 sind geschützte Markenbezeichnungen der ICI of America Inc., Stamford, Connecticut 06904.

Anstelle von 100,0 g $ZnSO_4.7H_2O$ kann man auch 50,0 g verwenden und im übrigen gleich vorgehen wie oben angegeben.

## Patentansprüche

1. Pharmazeutische Präparate zur topischen Behandlung von Virusinfektionen, gekennzeichnet durch einen Gehalt an einer antiviral wirksamen Kombination eines sulfatierten Polysaccharids oder Polymeren und Zinkionen in Form von dissoziierbaren Zinkverbindungen.

2. Pharmazeutische Präparate gemäss Anspruch 1, gekennzeichnet durch einen zusätzlichen Gehalt an Polyoxyäthlensorbitan-monolaurat und/oder -monooleat.

3. Pharmazeutische Präparate gemäss Anspruch 1, gekennzeichnet durch einen Gehalt an sulfatierten Polysacchariden oder Polymeren und Zinkionen in einem Mengenverhältnis von 1 mg zu 0,18 bis 18 mg.

4. Pharmazeutische Präparate gemäss Anspruch 3, gekennzeichnet durch einen Gehalt an Heparin und Zinkionen in einem Verhältnis von 160 IE zu 0,18 bis 18 mg.

5. Pharmazeutische Präparate gemäss Anspruch 1, gekennzeichnet durch einen Gehalt, pro g oder ml, von 0,1 bis 5 mg eines sulfatierten Polysaccharids oder Polymeren und 0,18 bis 18 mg Zinkionen.

6. Pharmazeutische Präparate gemäss Anspruch 1, gekennzeichnet durch einen Gehalt, pro g oder ml, von 16 bis 800 IE Heparin und 0,18 bis 18 mg Zinkionen.

7. Pharmazeutische Präparate gemäss Ansprüchen 5 und 6, denen die Zinkionen in Form von 0,8—80 mg $ZnSO_4.7H_2O$ als dissoziierbare Zinkverbindung zugefügt sind.

8. Pharmazeutische Präparate gemäss Anspruch 6, gekennzeichnet durch einen Gehalt, pro g oder ml, von 80—320 IE eines pharmazeutisch annehmbaren Salzes von Heparin und 0,45 bis 4,5 mg Zinkionen.

9. Pharmazeutische Präparate gemäss Ansprüchen 5 bis 8, gekennzeichnet durch einen zusätzlichen Gehalt pro g oder ml, von 0,5 bis 10 mg Polyoxyäthylensorbitanmonolaurat und/oder -monooleat.

## Revendications

1. Compositions pharmaceutiques pour le traitement topique des infections à virus, caractérisées en ce qu'elles contiennent une combinaison antivirale active d'un polysaccharide ou polymère sulfaté et d'ions zinc sous la forme de composés dissociables du zinc.

2. Compositions pharmaceutiques selon la revendication 1, caractérisées en ce qu'elles contiennent en outre du monolaurate et/ou du monooléate de sorbitanne polyoxyéthyléné.

3. Compositions pharmaceutiques selon la revendication 1, caractérisées en ce qu'elles contiennent des polysaccharides ou polymères sulfatés et des ions zinc dans des proportions relatives de 1 mg pour 0,18 à 18 mg.

4. Compositions pharmaceutiques selon la revendication 3, caractérisées en ce qu'elles contiennent de l'héparine et des ions zinc dans des proportions relatives de 160 UI pour 0,18 à 18 mg.

5. Compositions pharmaceutiques selon la revendication 1, caractérisées en ce qu'elles contiennent, par g ou ml, de 0,1 à 5 mg d'un polysaccharide ou polymère sulfaté et de 0,18 à 18 mg d'ions zinc.

6. Compositions pharmaceutiques selon la revendication 1, caractérisées en ce qu'elles contiennent, par g ou ml, de 16 à 800 UI d'héparine et de 0,18 à 18 mg d'ions zinc.

7. Compositions pharmaceutiques selon les revendications 5 et 6, dans lesquelles les ions zinc ont été introduits sous la forme de 0,8 à 80 mg de $ZnSO_4.7H_2O$ en tant que composé dissociable du zinc.

8. Compositions pharmaceutiques selon la revendication 6, caractérisées en ce qu'elles contiennent, par g ou ml, de 80 à 320 UI d'un sel acceptable pour l'usage pharmaceutique de l'héparine et de 0,45 à 4,5 mg d'ions zinc.

9. Compositions pharmaceutiques selon les revendications 5 à 8, caractérisées en ce qu'elles contiennent en outre, par g ou ml, de 0,5 à 10 mg de monolaurate ou de monooléate de sorbitanne polyoxyéthyléné.

**Claims**

1. A pharmaceutical preparation for the topical treatment of virus infections, characterised in that it contains an antivirally effective combination of a sulfated polysaccharide or polymer and zinc ions in the form of dissociable zinc compounds.

2. A pharmaceutical preparation according to claim 1, characterised in that it additionally contains polyoxyethylene sorbitan monolaurate and/or polyoxyethylene sorbitan monooleate.

3. A pharmaceutical preparation according to claim 1, characterised in that it contains sulfated polysaccharides or polymers and zinc ions in a ratio of 1 mg to 0.18 to 18 mg.

4. A pharmaceutical preparation according to claim 3, characterised in that it contains heparin and zinc ions in a ratio of 160 IU to 0.18 to 18 mg.

5. A pharmaceutical preparation according to claim 1, characterised in that it contains, per gram or millilitre, from 0.1 to 5 mg of a sulfated polysaccharide or polymer and 0.18 to 18 mg of zinc ions.

6. A pharmaceutical preparation according to claim 1, characterised in that it contains, per gram or millilitre, from 16 to 800 IU of heparin and 0.18 to 18 mg of zinc ions.

7. A pharmaceutical preparation according to one of claims 5 and 6, to which the zinc ions are added in the form of 0.8 to 80 mg of $ZnSO_4.7H_2O$ as dissociable zinc compound.

8. A pharmaceutical preparation according to claim 6, characterised in that it contains, per gram or millilitre, from 80 to 320 IU of a pharmaceutically acceptable salt of heparin and 0.45 to 4.5 mg of zinc ions.

9. A pharmaceutical preparation according to one of claims 5 to 8, characterised in that it additionally contains, per gram or millilitre, from 0.5 to 10 mg of polyoxyethylene sorbitan monolaurate and/or polyoxyethylene sorbitan monooleate.